# EUROPEAN PATENT APPLICATION

(11) **EP 1 728 513 A2**
(43) Date of publication of application: **06.12.2006**
(21) Application number: 06252644.7
(22) Date of filing: 19.05.2006
(51) Int. Cl.: A61K 31/4545, A61K 31/445, A61K 45/06, A61K 31/185, A61K 31/198

(54) **Stable desloratadine compositions**

(30) Priority: 20.05.2005 IN CH06082005; 20.09.2005 US 718789 P
(71) Applicant: Dr. Reddy's Laboratories, Inc., Bridgewater, NJ 08807 (US)
(72) Inventor: Agarwal, Sudeep Kumar, Hyderabad 500 072 A.P. (IN); Alagarsamy, Alagumurugan, Hyderabad 500 072 A.P. (IN); Nasare, Vijay Dinanathji, Anil Nasare, Nagpur Katol 441 302 Maharashtra (IN); Mohan, Mailatur Sivaraman, Kukatpally Hyderabad 500 072 A.P. (IN)
(74) Representative: Bates, Philip Ian

(57) **Abstract**

The present invention relates to stable pharmaceutical compositions of desloratadine comprising desloratadine in combination with a desloratadine-stabilizing amount of at least one amino acid.

## Description

### INTRODUCTION TO THE INVENTION

The present invention relates to stable pharmaceutical compositions of desloratadine (also known as "descarbonylethoxyloratadine") or pharmaceutically acceptable salts, solvates, polymorphs, enantiomers or mixtures thereof and processes for preparing the same.

Desloratadine (Formula I), is chemically named 8-chloro-6, 11-dihydro-11-(4-piperidinylidene)-5*H*-benzo[5,6] cyclohepta [1,2-*b*] pyridine and is a metabolic derivative of loratadine. It is used as a non-sedating antihistaminic agent and is commercially available as CLARINEX® film-coated tablets (5 mg) manufactured by Schering Corporation.

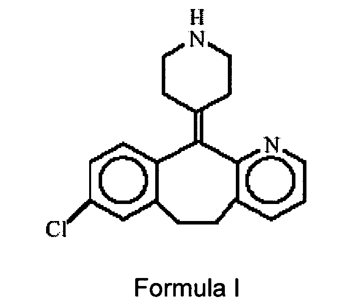

Desloratadine and its compositions are prone to oxidation and decomposition by acidic excipients to form impurities such as deschlorodesloratadine, dehydrodesloratadine and N-formyldesloratadine.

U.S. Patent No. 6,100,274 describes desloratadine (called "DCL" in the patent) and its pharmaceutical compositions comprising desloratadine and a desloratadine-protective amount of a pharmaceutically acceptable basic salt such as calcium dibasic phosphate and at least one disintegrant. The patent also discloses that desloratadine undergoes extensive degradation in the presence of common excipients such as lactose and stearic acid to form N-formyldesloratadine as a major degradation product. The basic salts of calcium, magnesium, or aluminum, plus avoidance of lactose and stearic acid, are used to control the degradation of desloratadine in pharmaceutical compositions.

The commercially available CLARINEX 5 mg desloratadine tablets contain the excipients dibasic calcium phosphate dihydrate USP, microcrystalline cellulose NF, corn starch NF, talc USP, carnauba wax NF, white wax NF, and a coating material consisting of lactose monohydrate, hydroxypropyl methylcellulose, titanium dioxide, polyethylene glycol, and FD&C Blue #2 aluminum lake.

U.S. Patent Application Publication No. 2002/0123504 reports the reactivity between lactose and desloratadine. Stable desloratadine-containing formulations are described as being free of mono- and di-saccharides, non-hygroscopic, and anhydrous.

International Application Publication No. WO 2005/065047 describes stable oral compositions of desloratadine and a stabilizer selected from the group comprising an antioxidant, a pharmaceutically acceptable organic compound that provides an alkaline pH, an alkali metal salt, or mixtures thereof, and pharmaceutically acceptable excipients.

Since desloratadine undergoes extensive degradation in presence of excipients such as lactose to form N-formyldesloratadine as a major degradation product, the use of an effective stabilizer to enhance the stability of the composition would be a significant improvement in the field of solid oral therapeutic compositions.

### SUMMARY OF THE INVENTION

The present invention relates to stable pharmaceutical compositions of desloratadine (descarbonylethoxyloratadine) or pharmaceutically acceptable salts, solvates, polymorphs, enantiomers or mixtures thereof and processes for preparing the same.

The present invention also relates to stable pharmaceutical compositions of desloratadine comprising desloratadine in combination with a desloratadine-stabilizing amount of at least one amino acid.

In another aspect the invention provides stable pharmaceutical compositions of desloratadine with improved stability of the composition having less than 0.5 percent by weight of the N-formyldesloratadine impurity.

In an embodiment, the invention includes a composition comprising desloratadine and an amino acid.

In another embodiment, the invention includes a pharmaceutical composition comprising desloratadine, at least one amino acid, and optionally a mono- or di-saccharide.

### BRIEF DESCRIPTION OF THE DRAWINGS

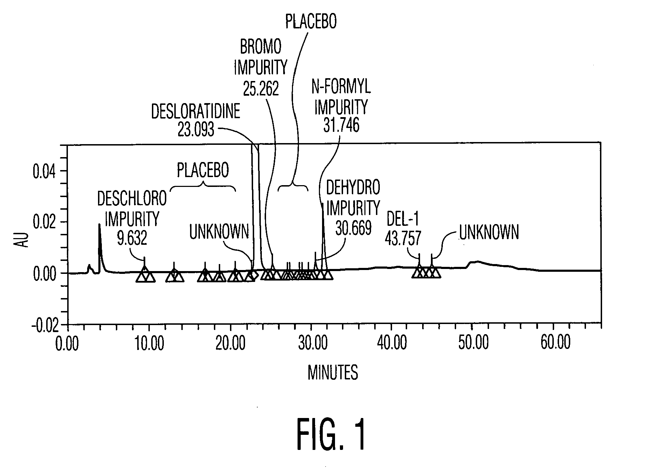

Figure 1 shows an HPLC chromatogram with peaks for desloratadine and the impurities.

### DETAILED DESCRIPTION OF THE INVENTION

The present invention relates to stable pharmaceutical compositions of desloratadine (descarbonylethoxyloratadine) or pharmaceutically acceptable salts, solvates, polymorphs, enantiomers or mixtures thereof and processes for preparing the same.

The present invention also relates to stable pharmaceutical compositions of desloratadine comprising desloratadine in combination with a desloratadine-stabilizing amount of at least one amino acid.

Amino acids that may be used in the present invention include but are not limited to arginine, cysteine, tyrosine, histidine, lysine, tryptophan and the like. In an embodiment, L-arginine was found to be useful in the context of the invention. However, the invention is not dependent on using any particular optical isomer of an amino acid.

The weight ratio of the stabilizing amount of amino acid to the amount of active substance can be in the range from about 4:1 to about 1:4, or from about 2:1 to about 1:2. Frequently, the amino acid concentration will be at least the concentration of desloratadine.

In another aspect the invention provides stable pharmaceutical compositions of desloratadine with improved stability, the composition having less than about 0.5 percent by weight of the N-formyldesloratadine impurity. In the context of the invention, this manner of describing impurity content is intended to mean that the impurity (degradation product) constitutes no more than 0.5 percent by weight of the original desloratadine content. These impurity concentrations should not be interpreted as weight percentages of the entire desloratadine-containing dosage form.

Other potential impurities can result from degradation of desloratadine, including 8-Chloro-11- (4-piperidinylidene)-benzo [5,6]-cyclohepta [1,2-b] pyridine. Certain other impurities potentially can be formed in a synthesis of desloratadine and/or by degradation of desloratadine, and these include 1-(4-piperidinylidene)-6,11-dihydro-5H-benzo [5,6]-cyclohepta [1,2-b] pyridine, 8-Bromo-11- (4-piperidinylidene)-6,11-dihydro-5H-benzo [5,6]-cyclohepta [1,2-b] pyridine, and 8-Chloro-11-(1-carboethoxy-4-piperidinylidene)-6,11-dihydro-5H-benzo[5,6]-cyclohepta [1,2-b]pyridine.

In general, it is desired that no single impurity has a concentration greater than about 0.4 percent by weight, and the total of the impurities is less than about 2 percent by weight, of the desloratadine content.

Stability of pharmaceutical compositions may be defined as the capability of a particular dosage form, in a specified package, to maintain its physical, chemical, microbiological, therapeutic and toxicological specifications.

Stability of pharmaceutical compositions may be affected by several factors, including the stability of the active pharmaceutical ingredient (API), API-excipient incompatibility, and mode of packaging. Factors such as oxidation, moisture, heat, and light may initiate and/or accelerate the chemical interaction thereby degrading the composition. Frequently, pharmaceutical compositions are tested for stability by storage in an adverse environment, such as the widely used 40°C, 75 % relative humidity ("RH") accelerated stability testing conditions, then analyzing for impurities that might have formed and changes in physical properties.

Desloratadine has been reported to be comparatively unstable in compositions comprising excipients such as lactose, and other mono- or di-saccharides. Hence the formulator should focus critically in the selection of the excipients and stabilization of compositions containing desloratadine.

The present invention relates to the stable pharmaceutical compositions of desloratadine wherein desloratadine is in intimate admixture with a stabilizer and other pharmaceutically acceptable excipients, but not limited to, blended, granulated or compressed dosage forms, that provides stabilization to the desloratadine in the composition.

In context of the present invention, during the preparation of the pharmaceutical compositions of desloratadine into a finished dosage form, one or more pharmaceutically acceptable excipients may optionally be used.

The pharmaceutically acceptable excipients may include but are not limited to diluents such as microcrystalline cellulose (MCC), silicified MCC (e.g. Prosolv™ HD 90), microfine cellulose, lactose, starch, pregelatinized starch, mannitol, sorbitol, dextrates, dextrin, maltodextrin, dextrose, calcium carbonate, calcium sulfate, dibasic calcium phosphate dihydrate, tribasic calcium phosphate, magnesium carbonate, magnesium oxide and the like.

Binders found useful include but are not limited to acacia, guar gum, alginic acid, dextrin, maltodextrin, methylcellulose, ethyl cellulose, hydroxyethyl cellulose, hydroxypropyl cellulose (e.g. KLUCEL®), hydroxypropyl methylcellulose (e.g. METHOCEL®), carboxymethylcellulose sodium, povidone (various grades of KOLLIDON®, PLASDONE®), starch such as corn starch and the like.

Disintegrants found useful include but are not limited to carboxymethyl cellulose sodium (e.g. Ac-Di-Sol®, Primellose®), crospovidone (e.g. Kollidon®, Polyplasdone®, povidone K-30, polacrilin potassium, starch, pregelatinized starch, sodium starch glycolate (e.g. Explotab® and the like.

Plasticizers that may be used include, without limitation, acetyltributyl citrate, phosphate esters, phthalate esters, amides, mineral oils, fatty acids and esters, glycerin, triacetin or sugars, fatty alcohols, polyethylene glycol, ethers of polyethylene glycol, fatty alcohols such as cetostearyl alcohol, cetyl alcohol, stearyl alcohol, oleyl alcohol, myristyl alcohol and the like.

Solvents that may be used include but are not limited to: aqueous solvents such as water; organic volatile solvents such as acetaldehyde, acetone, benzene, carbon disulphide, carbon tetrachloride, 1,2 dichloroethane, dichloromethane, N, n-dimethylformamide, 1,4-dioxane, epichlorhydrin, ethyl acetate, ethanol, ethyl ether, ethylene glycol, 2-ethoxyethanol (acetate), formaldehyde, isopropanolol, methanol, methyl n-butyl ketone, methyl ethyl ketone, 2-methoxyethanol (acetate), perchloroethylene, toluene, 1,1,1-trichloroethane, trichloroethylene; and the like.

Pharmaceutical compositions of the present invention may further include other ingredients, such as but not limited to pharmaceutically acceptable surfactants, glidants, lubricants, opacifiers, colorants and other commonly used excipients.

The pharmaceutical compositions may also be formulated in the form of:
pellets (extruded or fluidized) or spheres or cores that are either encapsulated as capsules or compressed into tablets or minitablets, which further can be filled into capsules; lyophilized powders filled into sachets or capsules; liquid formulations such as syrups, suspensions, emulsions; and the like.

The processes for manufacturing the formulations of the present invention are not limited to the processes described in the application and the formulation can be prepared by using any of the processes known to one skilled in the art.

The active ingredient along with stabilizer(s) can be granulated by wet granulation or dry granulation with or without excipients. The granules are prepared by sifting the active(s) and excipients through the desired mesh size sieve and then are mixed using a rapid mixer granulator or planetary mixer or mass mixer or ribbon mixer or fluid bed processor or any other suitable device. The blend can be granulated, such as by adding a solution of a binder whether aqueous or alcoholic or hydro-alcoholic in a low or high shear mixer, fluidized bed granulator and the like. The granulate can be dried using a tray drier or fluid bed drier or rotary cone vacuum drier and the like. The sizing of the granules can be done using an oscillating granulator or comminuting mill or any other conventional equipment equipped with a suitable screen. The dried granulate particles are sieved and then mixed with lubricants and disintegrants.

Alternatively the manufacture of granules may be done by direct compression by mixing the directly compressible excipients with active(s). The blend so obtained can either be compressed using a suitable device, such as a multi-station rotary machine to form compressed slugs or by roller compaction to form slugs, which are passed through a multimill, fluid energy mill, ball mill, colloid mill, roller mill, hammer mill and the like, equipped with a suitable screen. The milled slugs are then mixed with lubricants and disintegrants.

The tablets so obtained can further be optionally film coated. The coating can be done by techniques known to one skilled in the art such as spray coating, dip coating, fluidized bed coating and the like.

Various materials that may be used for coating include are but not limited to: hydrophilic materials such as carboxymethyl cellulose sodium, hydroxyethyl cellulose, hydroxypropyl methylcellulose (HPMC); homopolymers or copolymers of N-vinylpyrrolidone; vinyl and acrylic polymers; polyacrylic acid and the like; hydrophobic materials such as celluloses like ethyl cellulose, low substituted hydroxyl propyl cellulose (L-HPC), cellulose acetate, cellulose propionate (lower, medium or higher molecular weight), cellulose acetate propionate, cellulose acetate butyrate, cellulose acetate phthalate; polyalkyl methacrylates; polyalkyl acrylates; polyvinyl acetate (PVA); chitosan; crosslinked vinylpyrrolidone polymers; hydrogenated castor oil and the like. Other classes of rate controlling substances or their mixtures in various ratios as required are also within the purview of this invention without limitation.

In an aspect, solid oral dosage forms of the present invention can be formulated to provide a unit dose of desloratadine of about 1 to about 50, or about 2.5 to about 20, or about 5 to about 10, milligrams per day in single or divided doses.

The pharmaceutical compositions of the present invention are used in the treatment or prophylaxis of allergic rhinitis and other histamine-induced disorders.

The following examples will further describe certain specific aspects and embodiments of the invention in greater detail and are not intended to limit the scope of the invention.

### COMPARATIVE EXAMPLE: Composition with desloratadine and the reactive excipient lactose.

| **Ingredients** | **g/Batch of 5,000 Tablets** |
|---|---|
| Desloratadine | 25 |
| Corn starch | 387.5 |
| Corn starch (for binder preparation) | 25 |
| Water | 175 ml |
| Lactose monohydrate | 50 |
| Colloidal silicon dioxide | 5 |
| PEG 6000 | 7.5 |

The composition was prepared in a manner similar to that of following Example 1, but without L-arginine and in step 5 colloidal silicon dioxide, lactose and PEG 6000 were passed through an ASTM mesh #60 sieve and blended with the material of step 4 in a double cone blender for 5 minutes. The tablets were uncoated.

### EXAMPLE 1: Composition with L-arginine (Desloratadine:Arginine = 1:1).

| **Ingredients** | **g/Batch of 65,000 Tablets** |
|---|---|
| Desloratadine | 325 |
| L-arginine | 325 |
| Corn starch | 5362 |
| Corn starch (for binder preparation) | 325 |
| Water | 2990 ml |
| Colloidal silicon dioxide | 65 |
| PEG 6000 | 97 |
| Opadry Blue 03B50680* | 130 |

| | |
|---|---|
| * Opadry Blue is a prepared film coating material from Colorcon, West Point, Pennsyivania U.S.A., that contains hypromellose, titanium dioxide, macrogol, and FD&C Blue #2/indigo carmine aluminum lake. | |

### Manufacturing process:

1. Desloratadine, L-arginine and the larger amount of corn starch were sifted through an ASTM mesh #40 sieve and mixed for 10 minutes in a rapid mixer granulator (RMG).
2. Starch paste binder was prepared using cornstarch and water.
3. Mixture of step I was granulated using the starch paste of step 2.
4. The granulated mass was dried in fluid bed drier at a temperature of 65°C, until the moisture content of the granules was below 10% by weight, as tested using an infrared moisture balance, and finally sifted through an ASTM mesh #20 sieve. The retained particles were milled through a 1.5 mm sieve at medium speed, knives forward, and again sifted through an ASTM mesh #20 sieve.
5. Colloidal silicon dioxide and PEG 6000 were passed through an ASTM mesh #60 sieve and blended with the material of step 4 in a double cone blender for 5 minutes.
6. The blend was compressed into tablets using 6.35 mm round tooling and hardness of 3-8 kp (kp is "kilopond," a unit of force also called a kilogram of force, 1 kp = 1 kgf).
7. The tablets of step 6 were coated with a 14.5% w/w dispersion of Opadry Blue 03B50680 in water using an automated coating machine and after completion of coating process tablets were dried for 15 minutes at an inlet air temperature of 45 °C while jogging the pan.

### EXAMPLE 2: Composition with L-arginine (Desloratadine:Arginine = 1:2).

| **Ingredients** | **g/Batch of 4,000 Tablets** |
|---|---|
| Desloratadine | 20 |
| L-arginine | 40 |
| Corn starch | 310 |
| Corn starch (for binder preparation) | 20 |
| Water | 150 ml |
| Colloidal silicon dioxide | 4 |
| PEG 6000 | 6 |

The composition was prepared in a similar manner as for Example 1 except that the tablets were uncoated.

### EXAMPLE 3: Composition with L-arginine, desloratadine and the reactive excipient lactose.

| **Ingredients** | **g/Batch of 5,000 Tablets** |
|---|---|
| Desloratadine | 25 |
| L-arginine | 25 |
| Corn starch | 362.5 |
| Corn starch (for binder preparation) | 25 |
| Water | 179 ml |
| Lactose monohydrate | 50 |
| Colloidal silicon dioxide | 5 |
| PEG 6000 | 7.5 |

The composition was prepared in similar manner as Example 1 except that the tablets were uncoated and in step 5, colloidal silicon dioxide, lactose and PEG 6000 were passed through an ASTM mesh #60 sieve and blended with the material of step 4 in a double cone blender for 5 minutes.

### EXAMPLE 4: Comparative stability data of the composition of Example 1 and commercial CLARINEX® tablets.

% N-formyldesloratadine content after 4 weeks direct exposure to 40°C and 75% RH:

| **Example 1** | **CLARINEX® Tablets** |
|---|---|
| | (Batch No. 3STB090) |
| 0.066% | 1.207% |

### EXAMPLE 5: Stability study of compositions of Examples 1 and 2.

| **Time and Condition** | **N-Formyldesloratadine (%)** | |
|---|---|---|
| | **Example 1** | **Example 2** |
| Initial | 0.019 | 0.015 |
| 4 weeks at 40°C/75% RH; Direct exposure | 0.066 | -- |
| 4 weeks at 50°C; Direct exposure | 0.124** | 0.006* |

| | | |
|---|---|---|
| * 2 weeks data ** 60°C data | | |

### EXAMPLE 6: Stability study of desloratadine tablets with and without L-arginine in presence of the reactive excipient lactose.

| **Time and Condition** | **N-Formyldesloratadine (%)** | |
|---|---|---|
| | **Example 3** | **Comparative Example** |
| Initial | 0.052 | 0.038 |
| 4 weeks at 40°C/75% RH; Direct exposure | 0.375 | 2.679 |

These data show that desloratadine in the presence of lactose (Comparative Example) gives more N-formyl desloratadine impurity during storage, but the presence of the stabilizer (Example 3) L-arginine inhibits the

### formation of the impurity in the presence of the reactive excipient lactose.

EXAMPLE 7: Compositions of desloratadine prepared using anhydrous lactose.

| **Ingredients** | **kg/Batch of 500,000 Tablets** |
|---|---|
| Desloratadine | 2.5 |
| L-arginine | 2.75 |
| Corn starch | 41.25 |
| Corn starch (for binder preparation) | 2.5 |
| Water | 20 |
| Lactose anhydrous | 2.5 |
| Colloidal silicon dioxide | 0.5 |
| PEG 6000 | 0.75 |

| **Coating composition** | |
|---|---|
| Opadry Blue 03B50680 | 1.5 |
| Water | 9.2 |

The composition was prepared in a similar manner as for Example 1 except that in step 5 colloidal silicon dioxide, lactose and PEG 6000 were passed through an ASTM mesh #60 sieve and blended with the material of step 4 in a double cone blender for 5 minutes.

### EXAMPLE 8: Stability studies of the composition of Example 7.

| **Storage condition** | **N-formyldesloratadine (%)** | | | |
|---|---|---|---|---|
| | **Initial** | **1 month** | **2 months** | **3 months** |
| 40°C/75% RH; HDPE container (40 cc) with child resistant plastic cap | 0.008 | 0.05 | 0.1 | 0.23 |
| 40°C/75% RH; HDPE container (85 cc) with ribbed smooth plastic cap | 0.008 | 0.04 | 0.07 | 0.09 |
| 40°C/75% RH; Paper backed aluminium foil and cold formable foil as base | 0.008 | 0.04 | 0.07 | 0.1 |
| 40°C/75% RH; Paper backed aluminium foil and PVC film with Aclar as base | 0.008 | 0.05 | 0.1 | 0.14 |
| 25°C/60%RH; HDPE container (40 cc) with child resistant plastic cap | 0.008 | - | - | 0.02 |
| 25°C/60%RH; HDPE container (85 cc) with ribbed smooth plastic cap | 0.008 | - | - | 0.02 |
| 25°C/60%RH; Paper backed aluminium foil and cold formable foil as base | 0.008 | - | - | 0.02 |
| 25°C/60%RH; Paper backed aluminium foil and PVC film with Aclar as base | 0.008 | - | - | 0.02 |

| **Storage condition** | **Total Desloratadine Degradants (%)** | | | |
|---|---|---|---|---|
| | **Initial** | **1 month** | **2 months** | **3 months** |
| 40°C/75% RH; HDPE container (40 cc) with child resistant plastic cap | 0.16 | 0.17 | 0.21 | 0.36 |
| 40°C/75% RH; HDPE container (85 cc) with ribbed smooth plastic cap | 0.16 | 0.15 | 0.14 | 0.15 |
| 40°C/75% RH; Paper backed aluminium foil and cold formable foil as base | 0.16 | 0.17 | 0.16 | 0.17 |
| 40°C/75% RH; Paper backed aluminium foil and PVC film with Aclar as base | 0.16 | 0.16 | 0.2 | 0.23 |
| 25°C/60%RH; HDPE container (40 cc) with child resistant plastic cap | 0.16 | - | - | 0.09 |
| 25°C/60%RH; HDPE container (85 cc) with ribbed smooth plastic cap | 0.16 | - | - | 0.08 |
| 25°C/60%RH; Paper backed aluminium foil and cold formable foil as base | 0.16 | - | - | 0.07 |
| 25°C/60%RH; Paper backed aluminium foil and PVC film with Aclar as base | 0.16 | - | - | 0.06 |

### EXAMPLE 9: Compositions with L-Lysine, desloratadine and the reactive excipient lactose.

| **Ingredients** | **g/Batch of 5,000 Tablets** |
|---|---|
| Desloratadine | 25 |
| L-lysine | 25 |
| Corn starch | 362.5 |
| Corn starch (for binder preparation) | 25 |
| Water | 179 ml |
| Lactose monohydrate | 50 |
| Colloidal silicon dioxide | 5 |
| PEG 6000 | 7.5 |

The composition is prepared in a similar manner as in Example 3 except that L-arginine is replaced by L-lysine, and in step 5 colloidal silicon dioxide, lactose and PEG 6000 are passed through an ASTM mesh #60 sieve and blended with the material of step 4 in a double cone blender for 5 minutes.

### EXAMPLE 10: Composition with L-histidine, desloratadine and the reactive excipient lactose.

| **Ingredients** | **g/Batch of 5,000 Tablets** |
|---|---|
| Desloratadine | 25 |
| L-histidine | 75 |
| Corn starch | 312.5 |
| Corn starch (for binder preparation) | 25 |
| Water | 179 ml |
| Lactose monohydrate | 50 |
| Colloidal silicon dioxide | 5 |
| PEG 6000 | 7.5 |

The composition is prepared in a similar manner as in Example 3 except that L-arginine is replaced by L-histidine, and in step 5 colloidal silicon dioxide, lactose and PEG 6000 are passed through an ASTM mesh #60 sieve and blended with the material of step 4 in a double cone blender for 5 minutes.

### EXAMPLE 11: Composition with L-tryptophan, desloratadine and the reactive excipient lactose.

| **Ingredients** | **g/Batch of 5,000 Tablets** |
|---|---|
| Desloratadine | 25 |
| L-tryptophan | 75 |
| Corn starch | 312.5 |
| Corn starch (for binder preparation) | 25 |
| Water | 179 ml |
| Lactose monohydrate | 50 |
| Colloidal silicon dioxide | 5 |
| PEG 6000 | 7.5 |

The composition is prepared in a similar manner as in Example 3 except that L-arginine is replaced by L-tryptophan, and in step 5 colloidal silicon dioxide, lactose and PEG 6000 are passed through an ASTM mesh #60 sieve and blended with the material of step 4 in a double cone blender for 5 minutes.

### EXAMPLE 12: HPLC Chromatographic analysis procedure for tablets.

Preparation of sample: 40 tablets were taken and crushed. 50 mg was transferred to a 100 ml volumetric flask to which 70 ml of mobile phase was added. This dispersion was sonicated for 20 minutes and was finally diluted with mobile phase to the 100 ml mark. 10 ml of this solution was further centrifuged at 4000 rpm for 10 minutes and was finally filtered through a 0.45 µm Nylon 66 filter manufactured by Varian, Inc.

Chromatographic system: A liquid chromatograph equipped with a 280-nm UV detector was used. The column (4.6mm×250mm) contained a packing of octyl silane chemically bonded to porous silica or ceramic microparticles of 5 µm diameter. The column was operated at a temperature of 30 °C and flow rate of 1 ml/min. The injection volume was 40 µL. Run time was 65 minutes.

Mobile phase composition was a Phosphate buffer (pH 2.5):Methanol: Acetonitrile ratio of 16:3:1.

| **Impurity** | **Relative Retention Time** |
|---|---|
| Deschloro impurity: (11-(4-piperidinylidene)-6,11-dihydro-5H-benzo[5,6] cyclohepta[1,2-b] pyridine) | 0.42 |
| Bromo impurity: (8-Bromom-11-(4-piperidinylidene)-6,11-dihydro-5H-benzo[5,6] cyclohepta[1,2-b] pyridine) | 1.09 |
| Dehydro impurity: (8-chloro-11-(4-piperidinylidene) benzo[5,6] cyclohepta[1,2-b] pyridine) | 1.33 |
| N-formyl impurity | 1.37 |
| DEL-1 impurity: (8-chloro-11-(1-carboethoxy-4-piperidinylidene)-6,11-dihydro-5H-benzo[5,6] cyclohepta[1,2-b] pyridine | 1.9 |

Relative retention times are based on assigning a value of 1 to the desloratadine retention time.

An HPLC chromatogram showing the peaks for desloratadine and the impurities mentioned in the above table is present as Fig. 1, where "AU" is absorbance units from the detector. "Placebo" peaks as shown in the figure were identified from analysis of a blend of tablet excipients without desloratadine.

## Claims

1. A composition comprising desloratadine and an amino acid.

2. The composition of claim 1, further comprising a mono- or di-saccharide.

3. The composition of claim 2, wherein a mono- or di-saccharide comprises lactose.

4. The composition of claim 1, wherein an amino acid comprises arginine.

5. The composition of claim 1, wherein a weight ratio of amino acid to desloratadine is about 4:1 to about 1:4.

6. The composition of claim 1, wherein a weight ratio of amino acid to desloratadine is about 2:1 to about 1:2.

7. The composition of claim 1, wherein the amino acid concentration is at least equal to the desloratadine concentration.

8. The composition of claim 1, containing less than about 0.5 percent N-formyldesloratadine.

9. The composition of claim 1, containing less than about 0.4 percent N-formyldesloratadine.

10. A pharmaceutical composition comprising desloratadine, at least one amino acid, and optionally a mono- or di-saccharide.

11. The pharmaceutical composition of claim 10, comprising a mono- or di-saccharide.

12. The pharmaceutical composition of claim 10, wherein a mono- or d-saccharide comprises lactose.

13. The pharmaceutical composition of claim 10, wherein an amino acid comprises arginine.

14. The pharmaceutical composition of claim 10, wherein the amino acid concentration is at least equal to the desloratadine concentration.

15. The pharmaceutical composition of claim 10, wherein a weight ratio of amino acid to desloratadine is about 4:1 to about 1:4.

16. The pharmaceutical composition of claim 10, wherein a weight ratio of amino acid to desloratadine is about 2:1 to about 1:2.

17. The pharmaceutical composition of claim 10, containing less than about 0.5 percent N-formyldesloratadine.

18. The pharmaceutical composition of claim 10, containing less than about 0.4 percent N-formyldesloratadine.
